# EUROPEAN PATENT SPECIFICATION

(11) **EP 1 844 004 B1**
(45) Date of publication and mention of the grant of the patent: **24.06.2009**
(21) Application number: 06709585.1
(22) Date of filing: 30.01.2006
(51) Int. Cl.: C07C 255/61, C07D 239/94, C07D 403/14, A61P 31/14, A61K 31/5377, A61K 31/517

(54) **QUINAZOLINE DERIVATES AS ANTIVIRAL AGENTS**
CHINAZOLINDERIVATE ALS ANTIVIRALE MITTEL
DÉRIVÉS DE QUINAZOLINE SERVANT D'AGENTS ANTIVIRAUX

(30) Priority: 31.01.2005 GB 0501964; 04.02.2005 US 649564 P; 05.04.2005 US 668456 P
(43) Date of publication of application: 17.10.2007
(73) Proprietor: Arrow Therapeutics Limited, London SE1 1DA (GB)
(72) Inventor: COCKERILL, George, Stuart, Arrow Therapeutics Ltd., London SE11DA (GB); FLACK, Stephen Sean, Arrow Therapeutics Limited, London SE11DA (GB); MATHEWS, Neil, Arrow Therapeutics Limited, London SE11DA (GB); SALTER, James Iain, Arrow Therapeutics Limited, London SE11DA (GB)
(74) Representative: Srinivasan, Ravi Chandran
(86) International application number: PCT/GB2006/000294
(87) International publication number: WO 2006/079833

(56) References cited:
- WO-A-00/04900
- WO-A-20/05105761

## Description

The present invention relates to a series of quinazoline derivatives which are useful in treating or preventing a flaviviridae infection.

Viruses of the family flaviviridae are small, icosahedral, enveloped viruses that contain a positive-sense RNA genome. The family consists of three genera, flavivirus, pestivirus and hepacivirus.

Many of the flaviviridae viruses are important human pathogens. Indeed, the hepacivirus genus includes the hepatitis C virus. However, there exists, as yet, no effective and safe treatment for flaviviridae infections.

WO 00/04900 discloses benzimidazole antiviral compounds which contain aromatic substituents.

It has now surprisingly been found that the quinazoline derivatives of the formula (I) are active in inhibiting replication of flaviviridae viruses and are therefore effective in treating or preventing a flaviviridae infection. The present invention therefore provides a quinazoline derivative of formula (I), or a pharmaceutically acceptable salt thereof, 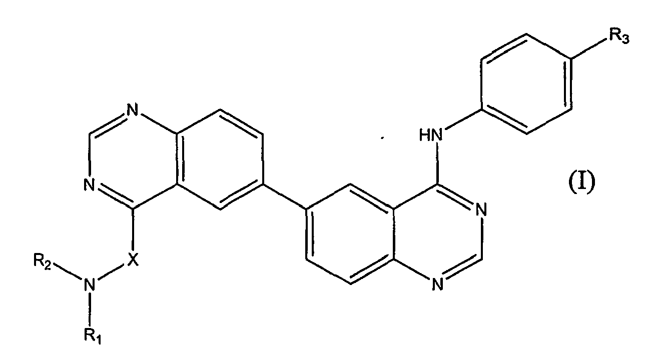 wherein:
- X represents a direct bond or a moiety -L-NR-, wherein R is hydrogen or C₁-C₄ alkyl, and L represents a C₁-C₄ alkylene, C₆-C₁₀ aryl or 5- to 10- membered heteroaryl moiety;
- either R₁ and R₂, together with the N atom to which they are attached, form a 5-to 10- membered heterocyclyl group or a 5- to 10- membered heteroaryl group, or R₁ represents hydrogen, C₆-C₁₀ aryl, C₁-C₄ alkyl or C₁-C₄ hydroxyalkyl and R₂ represents C₆-C₁₀ aryl, C₁-C₄ alkyl or C₁-C₄ hydroxyalkyl; and
- R₃ represents a C₆-C₁₀ aryl, C₃-C₆ carbocyclyl, 5- to 10- membered heteroaryl or 5- to 10- membered heterocyclyl moiety,
said aryl, carbocyclyl, heteroaryl and heterocyclyl groups being unsubstituted or substituted by 1, 2 or 3 substituents selected from halogen, C₁-C₄ alkyl, C₁-C₄ alkoxy, C₁-C₄ haloallcyl, C₁-C₄ haloalkoxy, hydroxy, thiol, -NH₂, C₁-C₄ hydroxyalkyl, C₁-C₄ thioalkyl and C₁-C₄ aminoalkyl substituents.

Typically, the compound of formula (I) is a compound of formula (I') 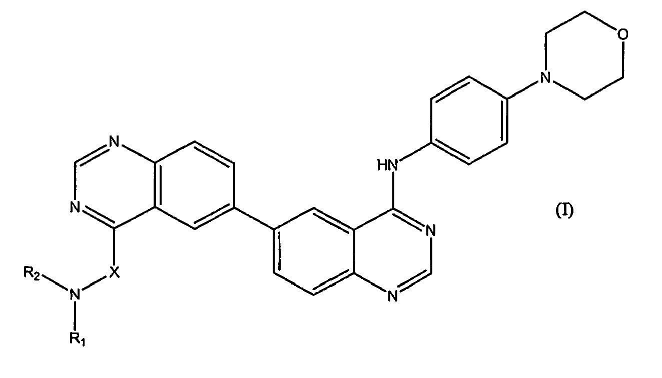 wherein:
- X represents a direct bond or a moiety -L-NR-, wherein R is hydrogen or C₁-C₄ alkyl, and L represents a C₁-C₄ alkylene, C₆-C₁₀ aryl or 5- to 10- membered heteroaryl moiety; and
- either R₁ and R₂ are the same or different and each represent a C₁-C₄ alkyl or C₁-C₄ hydroxyalkyl group or R₁ and R₂, together with the N atom to which they are attached, form a 5- to 10- membered heterocyclyl group,
said aryl, heteroaryl and heterocyclyl groups being unsubstituted or substituted by 1, 2 or 3 substituents selected from halogen, C₁-C₄ alkyl, C₁-C₄ alkoxy, C₁-C₄ haloalkyl, C₁-C₄ haloalkoxy, hydroxy, thiol, -NH₂, C₁-C₄ hydroxyalkyl, C₁-C₄ thioalkyl and C₁-C₄ aminoalkyl substituents.

For the avoidance of doubt, when X is -L-NR-, the NR moiety is attached to the quinazoline ring and the moiety L is attached to the -NR₁R₂ group.

As used herein, a C₁-C₄ alkyl group or moiety is a linear or branched alkyl group or moiety containing from 1 to 4 carbon atoms. Examples of C₁-C₄ alkyl groups and moieties include methyl, ethyl, n-propyl, i-propyl, n-butyl, i-butyl and t-butyl. For the avoidance of doubt, where two alkyl moieties are present, the alkyl moieties may be the same or different.

As used herein, a C₁-C₄ alkylene group or moiety is a linear or branched alkylene group or moiety. Examples include methylene, ethylene and n-propylene groups and moieties.

Typically, as used herein, a C₆-C₁₀ aryl group or moiety is phenyl or naphthyl. Phenyl is preferred.

As used herein, a halogen is typically chlorine, fluorine, bromine or iodine and is preferably chlorine, bromine or fluorine.

As used herein, a C₁-C₄ alkoxy group is typically a said C₁-C₄ alkyl group attached to an oxygen atom. A haloalkyl or haloalkoxy group is typically a said alkyl or alkoxy group substituted by one or more said halogen atoms. Typically, it is substituted by 1, 2 or 3 said halogen atoms. Preferred haloalkyl and haloalkoxy groups include perhaloalkyl and perhaloalkoxy groups such as -CX₃ and -OCX₃ wherein X is a said halogen atom, for example chlorine and fluorine. Particularly preferred haloalkyl groups are -CF₃ and -CCl₃. Particularly preferred haloalkoxy groups are -OCF₃ and -OCCl₃.

As used herein a C₁-C₄ hydroxyalkyl group is a C₁-C₄ alkyl group substituted by one or more hydroxy groups. Typically, it is substituted by one, two or three hydroxy groups. Preferably, it is substituted by a single hydroxy group. A preferred hydroxyalkyl group is -CH₂-OH.

As used herein, a C₁-C₄ thioalkyl group is a C₁-C₄ alkyl group substituted by one or more thio groups (-SH). Typically, it is substituted by one, two or three thio groups. Preferably, it is substituted by a single thio group.

As used herein, a C₁-C₄ aminoalkyl group is a C₁-C₄ alkyl group substituted by one or more -NH₂ groups. Typically, it is substituted by one, two or three -NH₂ groups. Preferably, it is substituted by a single -NH₂ group.

As used herein, a 5- to 10- membered heteroaryl group or moiety is a 5- to 10-membered aromatic ring, such as a 5- or 6- membered ring, containing at least one heteroatom, for example 1, 2 or 3 heteroatoms, selected from O, S and N.

Typically, a heteroaryl group or moiety is monocyclic. Examples of heteroaryl groups include pyridyl, pyrazinyl, pyrimidinyl, pyridazinyl, furanyl, thienyl, pyrazolidinyl, pyrrolyl, oxadiazolyl, oxazolyl, isoxazolyl, thiazolyl, thiadiazolyl, imidazolyl, triazolyl and pyrazolyl groups. Furanyl, triazolyl, thienyl, pyrimidinyl and thiazolyl groups are preferred. Triazolyl groups are particularly preferred.

As used herein, a 5- to 10- membered heterocyclyl group or moiety is a non-aromatic, saturated or unsaturated C₅-C₁₀ carbocyclic ring in which one or more, for example 1, 2 or 3, of the carbon atoms are replaced with a moiety selected from N, O, S, S(O) and S(O)₂. Typically, it is a 5- to 6- membered ring. Typically, a heterocyclyl group or moiety is monocyclic.

Preferably, a heterocyclyl group or moiety is a saturated C₅-C₆ cycloalkyl group in which 1 or 2 of the carbon atoms are replaced with a moiety selected from NH, O and S.

Suitable heterocyclyl groups and moieties include pyrazolidinyl, piperidyl, piperazinyl, thiomorpholinyl, S-oxo-thiomorpholinyl, S,S-dioxo-thiomorpholinyl, morpholinyl, pyrrolidinyl, pyrrolinyl, imidazolidinyl, imidazolinyl, 1,3-dioxolanyl, 1,4-dioxolyl and pyrazolinyl groups and moieties. Morpholino, pyrrolidinyl and piperazinyl groups are preferred.

As used herein, a C₃-C₆ carbocyclyl group is a non-aromatic saturated or unsaturated monocyclic hydrocarbon ring, having from 3 to 6 carbon atoms. Preferably it is a saturated hydrocarbon ring (i.e. a cycloalkyl group) having from 3 to 6 carbon atoms. Examples include cyclopropyl, cyclobutyl, cyclopentyl and cyclohexyl. It is preferably cyclopentyl or cyclohexyl.

Typically, the aryl, heteroaryl, carbocyclyl and heterocyclyl moieties in the R₁, R₂, R₃ and X moieties are unsubstituted or substituted by 1 or 2 substituents selected from halogen, C₁-C₄ alkyl and C₁-C₄ haloalkyl substituents. More preferably, they are unsubstituted.

Typically, R is H.

Typically, L is a phenylene moiety. More preferably, L is a 1,4-phenylene moiety.

Typically, X is a direct bond or -L-NR- wherein L and R are as defined above. Preferably, X is -L-NR-.

Preferably, when R₁ and R₂ do not together form a cyclic moiety, R₁ is hydrogen. Preferably, R₂ is C₆ to C₁₀ aryl, more preferably phenyl.

Preferably, when R₁ and R₂ together form a cyclic moiety, R₁ and R₂, together with the N atom to which they are attached, form a 5- to 6- membered heterocyclyl or 5-to 6- membered heteroaryl group. The heterocyclyl group is preferably a saturated 5- to 6- membered heterocycle, which contains 1 or 2 heteroatoms selected from N, O and S.

Most preferably, when R₁ and R₂ together form a cyclic moiety, R₁ and R₂, together with the N atom to which they are attached, form a morpholino group or a triazolyl group.

Preferably, R₃ represents a 5- to 6- membered heteroaryl or heterocyclyl moiety. More preferably, R₃ represents a morpholino or triazolyl moiety.

Preferred compounds of the invention are those in which:
- X represents a direct bond or -L-NR-, wherein R is hydrogen or C₁-C₄ alkyl, and L represents a phenylene moiety;
- either R₁ and R₂, together with the N atom to which they are attached, form a 5-to 6- membered heteroaryl or heterocyclyl group or R₁ represents hydrogen and R₂ represents a phenyl group; and
- R₃ represents a 5- to 6- membered heteroaryl or heterocyclyl moiety,
the phenyl, heterocyclyl and heteroaryl moieties being unsubstituted or substituted by 1 or 2 substituents selected from halogen, C₁-C₄ alkyl and C₁-C₄ haloalkyl substituents.

Typically, in these preferred compounds of the invention, R₁ and R₂, together with the N atom to which they are attached, form a saturated 5- to 6- membered heterocyclic group which contains 1 or 2 heteroatoms selected from N, O and S.

Typically, in these preferred compounds of the invention, R₃ represents a morpholino or triazolyl moiety.

Further preferred compounds of the invention are compounds of formula (I') in which:
- X represents -L-NR-, wherein R is hydrogen or C₁-C₄ alkyl, and L represents a phenylene moiety; and
- R₁ and R₂, together with the N atom to which they are attached, form a saturated 5- to 6- membered heterocycle which contains 1 or 2 heteroatoms selected from N, O and S,
the phenylene moiety and the heterocycle being unsubstituted or substituted by 1 or 2 substituents selected from halogen, C₁-C₄ alkyl and C₁-C₄ haloalkyl substituents.

Preferred compounds of formula (I) are
6-(4-(4-morpholinophenylamino)quinazolin-6-yl)-N,N-dimethylquinazolin-4-amine
6-(4-(4-morpholinophenylamino)quinazolin-6-yl)-N,N-diethylquinazolin-4-amine
6-(4-(4-morpholinophenylaniino)quinazolin-6-yl)-N-(2-(dimethylamino)ethyl)quinazolin-4-amine
6-(4-(4-morpholinophenylamino)quinazolin-6-yl)-N-(2-(pyrrolidin-1-yl)ethyl)quinazolin-4-amine
6-(4-(4-morpholinophenylamino)quinazolin-6-yl)-N-(2-morpholinoethyl)quinazolin-4-amine
6-(4-(4-morpholinophenylamino)quinazolin-6-yl)-N-(3-(pyrrolidin-1-yl)propyl)quinazolin-4-amine
6-(4-(4-morpholinophenylamino)quinazolin-6-yl)-N-(3-morpholinopropyl)quinazolin-4-amine
N-(4-morpholinophenyl)-6-(4-morpholinoquinazolin-6-yl)quinazolin-4-amine
N-(4-morpholinophenyl)-6-(4-(piperazin-1-yl)quinazolin-6-yl)quinazolin-4-amine
6-(4-(4-methylpiperazin-1-yl)quinazolin-6-yl)-N-(4-morpholinophenyl)quinazolin-4-amine
6-(4-(4-ethylpiperazin-1-yl)quinazolin-6-yl)-N-(4-morpholinophenyl)quinazolin-4-amine
6-(4-(2-(N-(2-hydroxyethyl)-N-methylamino)ethylamino)quinazolin-6-yl)-N-(4-morpholinophenyl)quinazolin-4-amine
N*4*-(4-Morpholin-4-yl-phenyl)-N*4*-phenyl-[6,6']biquinazolinyl-4,4'-diamine
N*4*,N*4'*-Bis-(4-[1,2,4]triazol-1-yl-phenyl)-[6,6']biquinazolinyl-4,4'-diamine

A particularly preferred compounds of formula (I) is:
N-(4-morpholinophenyl)-6-(4-(4-morpholinophenylamino)quinazolin-6-yl)quinazolin-4-amine. That compound has the structure:

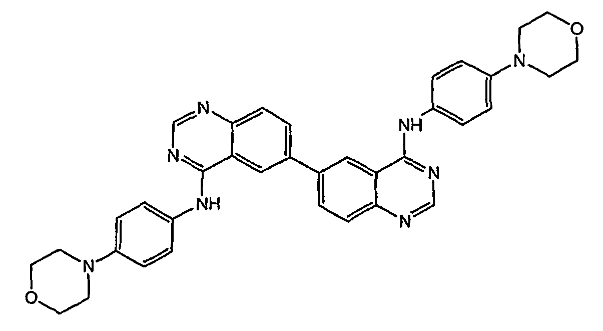

Compounds of formula (I) containing one or more chiral centre may be used in enantiomerically or diastereoisomerically pure form, or in the form of a mixture of isomers. For the avoidance of doubt, the compounds of formula (I) can, if desired, be used in the form of solvates. Further, for the avoidance of doubt, the compounds of the invention may be used in any tautomeric form.

As used herein, a pharmaceutically acceptable salt is a salt with a pharmaceutically acceptable acid or base. Pharmaceutically acceptable acids include both inorganic acids such as hydrochloric, sulphuric, phosphoric, diphosphoric, hydrobromic or nitric acid and organic acids such as citric, fumaric, maleic, malic, ascorbic, succinic, tartaric, benzoic, acetic, methanesulphonic, ethanesulphonic, benzenesulphonic orp-toluenesulphonic acid. Pharmaceutically acceptable bases include alkali metal (e.g. sodium or potassium) and alkali earth metal (e.g. calcium or magnesium) hydroxides and organic bases such as alkyl amines, aralkyl amines and heterocyclic amines. Preferred salts are hydrochloride salts. A particularly preferred salt is the tetrahydrochloride salt.

The compounds of the invention can, for example, be prepared according to the following reaction scheme.

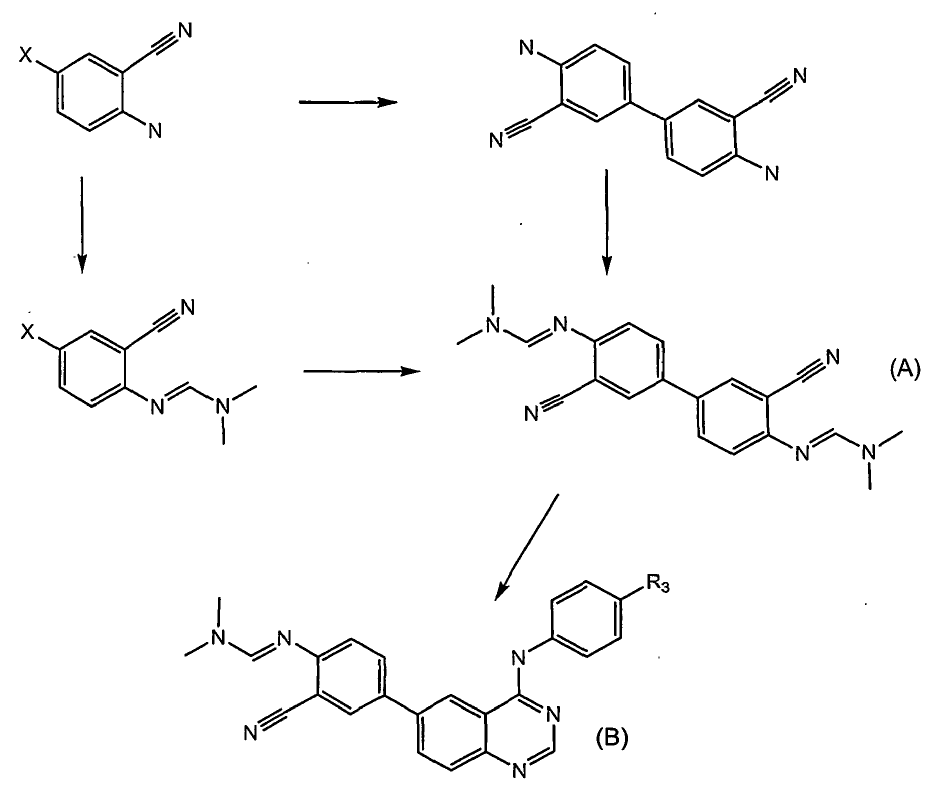

As depicted above, Intermediate A can be prepared by dimerisation of an appropriate aniline, or via dimerisation of an amidine. The latter route is preferred. X in the above reaction scheme is typically iodine or bromine. Dimerisation can be effected, for example, with Pd(dppf)Cl₂, DMSO/H₂O, K₃PO₄ and 4,4,5,5-tetramethyl-2-(4,4,5,5-tetramethyl-1,3,2-dioxaborolan-2-yl)-1,3,2-dioxaborolane. Intermediate A can be reacted with a sub-stoichiometric amount of a substituted aniline to produce intermediate B. Intermediate B can be reacted with a nucleophilic amine of formula R₁R₂N-X-H to yield the asymmetric quinazolinyl-quinazolines of the invention.

The intermediate B can also be prepared by transition metal-mediated coupling of a quinazoline iodide (C) with a suitable quinazoline or quinazoline precursor as shown below 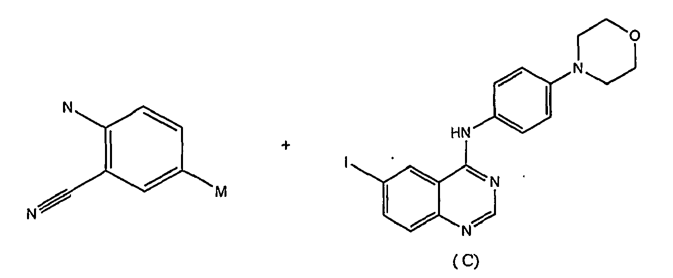 M in the above reaction scheme could be B(OR)₂ or SnR₃.

The intermediate B can also be prepared by transition metal-mediated coupling of aminobenzonitrile iodide (D) with a suitable quinazoline or quinazoline precursor as shown below. 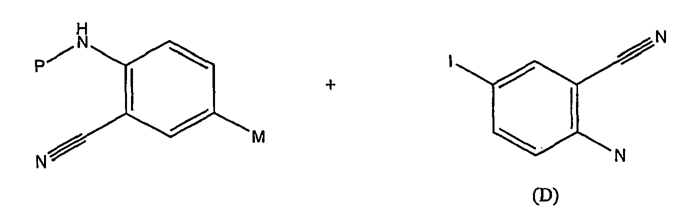 P in the above scheme is a suitable protecting group (eg N-tert butoxycarbonyl) so as to differentiate the aniline nitrogens in the resulting biphenyl product.

The compound *N*-(4-morpholinophenyl)-6-(4-(4-morpholinophenylamino)quinazolin-6-yl)quinazolin-4-amine can also be prepared by the following reaction.

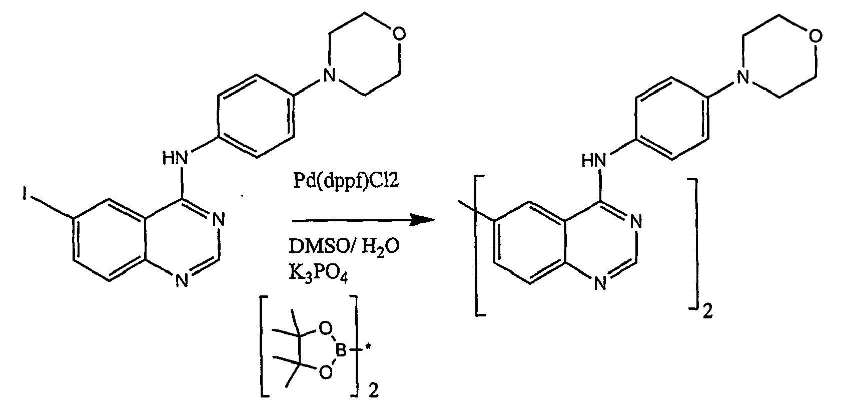

The compounds of the invention can be salified by standard techniques, in particular by reaction with an appropriate acid or base.

The intermediates A and B depicted above are believed to be novel, and accordingly form part of the invention.

The starting materials in the above reaction scheme are known compounds, or can be prepared by analogy with known methods.

The compounds of the present invention are therapeutically useful. The present invention therefore provides a quinazoline derivative of the formula (I), as defined above, or a pharmaceutically acceptable salt thereof, for use in treating the human or animal body. Also provided is a pharmaceutical composition comprising a quinazoline derivative of the formula (I), as defined above, or a pharmaceutically acceptable salt thereof, and a pharmaceutically acceptable carrier or diluent.

Said pharmaceutical composition typically contains up to 85 wt% of a compound of the invention. More typically, it contains up to 50 wt% of a compound of the invention. Preferred pharmaceutical compositions are sterile and pyrogen free. Further, the pharmaceutical compositions provided by the invention typically contain a compound of the invention which is a substantially pure optical isomer.

As explained above, the compounds of the invention are active against a flaviviridae infection. The present invention therefore provides the use of a quinazoline derivative of the formula (I), as defined above, or a pharmaceutically acceptable salt thereof, in the manufacture of a medicament for use in treating or preventing a flaviviridae infection. Also provided is a compound for treating a patient suffering from or susceptible to a flaviviridae infection, in a method which comprises administering to said patient an effective amount of a quinazoline derivative of formula (I) or a pharmaceutically acceptable salt thereof.

The flaviviridae family contains three genera. These are hepacivirus, flavivirus and pestivirus. The compounds of the invention are active in treating or preventing a hepacivirus infection, a flavivirus infection or a pestivirus infection.

Typical pestivirus infections which can be treated with the compounds of the invention include bovine viral diarrhea virus, classical swine fever virus and border disease virus.

Typical flavivirus infections which can be treated with the compounds of the invention include yellow fever virus, dengue fever virus, Japanese encephalitis virus and tick borne encephalitis virus.

Typical hepacivirus infections that can be treated with the compounds of the invention include hepatitis C virus.

Compounds of the present invention are especially active against hepatitis C. Typically, said flavivirus is therefore hepatitis C virus.

The compounds of the invention may be administered in a variety of dosage forms. Thus, they can be administered orally, for example as tablets, troches, lozenges, aqueous or oily suspensions, dispersible powders or granules. The compounds of the invention may also be administered parenterally, whether subcutaneously, intravenously, intramuscularly, intrasternally, transdermally or by infusion techniques. The compounds may also be administered as suppositories.

The compounds of the invention are typically formulated for administration with a pharmaceutically acceptable carrier or diluent. For example, solid oral forms may contain, together with the active compound, diluents, e.g. lactose, dextrose, saccharose, cellulose, corn starch or potato starch; lubricants, e.g. silica, talc, stearic acid, magnesium or calcium stearate, and/or polyethylene glycols; binding agents; e.g. starches, arabic gums, gelatin, methylcellulose, carboxymethylcellulose or polyvinyl pyrrolidone; disaggregating agents, e.g. starch, alginic acid, alginates or sodium starch glycolate; effervescing mixtures; dyestuffs; sweeteners; wetting agents, such as lecithin, polysorbates, laurylsulphates; and, in general, non toxic and pharmacologically inactive substances used in pharmaceutical formulations. Such pharmaceutical preparations may be manufactured in known manner, for example, by means of mixing, granulating, tableting, sugar coating, or film coating processes.

Liquid dispersions for oral administration may be syrups, emulsions and suspensions. The syrups may contain as carriers, for example, saccharose or saccharose with glycerine and/or mannitol and/or sorbitol.

Suspensions and emulsions may contain as carrier, for example a natural gum, agar, sodium alginate, pectin, methylcellulose, carboxymethylcellulose, or polyvinyl alcohol. The suspension or solutions for intramuscular injections may contain, together with the active compound, a pharmaceutically acceptable carrier, e.g. sterile water, olive oil, ethyl oleate, glycols, e.g. propylene glycol, and if desired, a suitable amount of lidocaine hydrochloride.

Solutions for injection or infusion may contain as carrier, for example, sterile water or preferably they may be in the form of sterile, aqueous, isotonic saline solutions.

Compounds of the present invention may be used in conjunction with known anti-viral agents. Preferred known anti-viral agents in this regard are interferon and ribavirin, and derivatives thereof, which are known for the treatment of hepatitis C (Clinical Microbiology Reviews, Jan. 2000, 67-82). The said medicament therefore typically further comprises interferon or a derivative thereof and/or ribavirin or a derivative thereof. Further, the present invention provides a pharmaceutical composition comprising:
(a) a quinazoline derivative of the formula (I), as defined above, or a pharmaceutically acceptable salt thereof;
(b) interferon or a derivative thereof and/or ribavirin or a derivative thereof; and
(c) a pharmaceutically acceptable carrier or diluent.

Also provided is a product comprising:
(a) a quinazoline derivative of the formula (I), as defined above, or a pharmaceutically acceptable salt thereof; and
(b) interferon or a derivative thereof and/or ribavirin or a derivative thereof,
for separate, simultaneous or sequential use in the treatment of the human or animal body.

A preferred interferon derivative is PEG-interferon. A preferred ribavirin derivative is viramidine.

A therapeutically effective amount of a compound of the invention is administered to a patient. A typical dose is from about 0.01 to 100 mg per kg of body weight, according to the activity of the specific compound, the age, weight and conditions of the subject to be treated, the type and severity of the disease and the frequency and route of administration. Preferably, daily dosage levels are from 0.05 to 16 mg per kg of body weight, more preferably, from 0.05 to 1.25 mg per kg of body weight.

The following Examples illustrate the invention. They do not however, limit the invention in any way. In this regard, it is important to understand that the particular assay used in the Examples section is designed only to provide an indication of antiviral activity. There are many assays available to determine such activity, and a negative result in any one particular assay is therefore not determinative.

### EXAMPLES

All temperatures are in °C. Thin layer chromatography (TLC) was carried out on Si 60G coated plastic plates with uv254 indicator (Polygram). All NMR spectra were obtained at 250MHz in d⁶-DMSO unless stated otherwise.

### LC-MS CONDITIONS

Samples were run on a MicroMass ZMD, using electrospray with simultaneous positive - negative ion detection.
Column : Synergi Hydro-RP, 30 x 4.6mm I.D, 4 µm.
Gradient: 95:5 to 5:95 v/v H₂O/CH₃CN + 0.05% Formic Acid over 4.0 min, hold 3 min, return to 95:5 v/v H₂O/CH₃C + 0.05% Formic Acid over 0.2 min and hold at 95:5 v/v H₂O/CH₃C + 0.05% Formic Acid over 3 min.
Detection: PDA 250 - 340 nm.
Flow rate : 1.5 ml/min

### Example 1: N-(4-morpholinophenyl)-6-(4-(4-morpholinophenylamino)quinazolin-6-yl)quinazolin-4-amine

A mixture of 6-iodo-*N*-(4-morpholinophenyl)quinazolin-4-amine (1eq, 1.004g), 4,4,5,5-tetramethyl-2-(4,4,5,5-tetramethyl-1,3,2-dioxaborolan-2-yl)-1,3,2-dioxaborolane (0.5eq, 295mg), dichloro diphenylphosphinylferrocene palladium (II) (10%, 200mg) and powdered potassium phosphate (3eq, 1.48g) in DMSO / H₂O (5:1, 12ml) was heated to 80 degrees for 1.5h. The cooled reaction mixture was diluted with water and the green ppt isolated by filtration to give the crude product (1.46g).

A portion of this material (517mg) was dissolved, with sonication and warming in aqueous 2N HCl (2.11ml, ∼5eq) and stirred whilst MeCN (21ml) added. Stirring and agitation was continued until the initial dark residue transformed into a solid. Filtration and drying in vacuo gave the title compound as a light brown solid (253mg, 83% recovery based on .4HCl)
LC-MS rt 2.41 m/z 611
1H NMR (DMSO) δ 11.80 (1H, brS), 9.71 (1H, s), 8.71 (1H, s), 8.52 (1H, d) 7.81 (1H, d), 7.57 (2H, d), 6.90 (2H, d), 4.29 (br s), 3.57 (4H, m), 2.98 (4H, m)
1H NMR (DMSO + K₂CO₃) δ 10.55 (1H, brS), 9.69 (1H, s), 8.66(1H, s), 8.58 (1H, d) 8.0 (2H, d), 7.12 (2H, d), 3.89 (4H, m), 3.24 (4H, m)
13C NMR (DMSO) δ 159.1, 151.0, 147.7, 138.1, 137.05, 134.1, 130.2, 125.8, 123.2, 120.25, 116.5, 114.2, 65.84, 49.82

### Alternative Synthesis of N-(4-morpholinophenyl)-6-(4-(4-morpholinophenylamino)quinazolin-6-yl)quinazolin-4-amine

### N'-(2-Cyano-4-iodo phenyl)-N,N-dimethyl-formamidirte:

2-Amino-5-iodo-benzonitrile (5g, 20.5mmol) was heated to reflux in DMF-DMA (20ml) for 2h. On cooling and concentrating to dryness *in vacuo* the residue was filtered through silica (20g, SiO2) eluting with dichloromethane to afford a brown oil (6.15g, 100%)
¹H NMR (CDCl₃ δ 7.71 (1H, d), 7.58 (1H, dd), 7.505 (1H, s), 6.64 (1H, d), 3.01 (6H, s)
LC-MS rt 3.46 m/z 299 MH+

### N'-[3,3'-Dicyano-4'-(dimethylamino-methyleneamino)-biphenyl-4-yl]-N,N-dimethylformamidine:

N'-(2-Cyano-4-iodo-phenyl)-N,N-dimethyl-formamidine (1.5g, 5mmol), bis(pinacolato)diboron (762mg, 0.6eq) in DMSO (15ml) and potassium phosphate (3.2g, 3 eq) in water (3ml, CARE exotherm) were mixed with stirring. Dichloro (diphenylphosphinylferrocenyl) palladium (II). DCM (204mg, 5mol%) was added and the mixture heated to 80° overnight. The cooled reaction mixture was partitioned between water (100ml) and DCM(150ml). The organic phase was separated and washed twice with water before being dried (Na2SO4) and concentrated to dryness. Trituration with diethyl ether afforded a brown crystalline solid (530mg, 62%)
¹H NMR (DMSO) δ 8.045 (1H, s), 7.98(1H, d), 7.86 (1H, dd), 7.24 (1H, d) 3.1 (3H, S) 3.02 ( 3H, s)
LC-MS rt 2.12 M/z 345

### N-(4-morpholilzophenyl)-6-(4-(4-morpholinophenylamino)quinazolin-6-yl)quinazolin-4-amine. Hydrochloride salt.

N'-[3,3'-Dicyano-4'-(dimethylamino-methyleneamino)-biphenyl-4-yl]-N,N-dimethylformamidine (35mg, 0.1mmol) and 4-morpholinoaniline (36mg, 0.2mmol) were combined in acetic acid (250uL) and heated to 85° for 90minutes. The mixture was diluted with 10% aq NaOH and the solid isolated by filtration and washed with water. The solid was then slurried in 2M HCl (1ml) to give a red solution that on addition of acetone precipitated the title compound hydrochloride salt as a light coloured solid. Filtration, washing with further acetone and drying in vacuo gave the title compound as a red brown solid (58mg, 77%)
LC-MS and 1H NMR as previously described.

### Example 2: N*4*-(4-Morpholin-4-yl-phenyl)-N*4*-phenyl-[6,6']biquinazolinyl-4,4'-diamine

### N'-(2-Cyano-4-iodo-phenyl)-N,N-dimethyl-formamidine

2-Amino-5-iodo-benzonitrile (5g) in DMF-DMA (10ml) was heated to 110° for 2h. The cooled reaction mixture was diluted with water (100ml) and extracted into ethyl acetate (3x100ml). The combined organic phases were dried (MgSO4) and concentrated to give a viscous brown oil (∼ quantitative) which was used without further purification.
¹H NMR (CDCl₃) δ 7.77 (1H, m), 7.65 (1H, dm, J8.85Hz), 7.57 (1H, s), 6.70 (1H, d, J8.85Hz), 3.012 (6H, s)
LC-MS rt 3.46 m/z 299.78

### 3-Cyano-4-(dimethylamino-methyleneamino)-phenylboronic acid

N'-(2-Cyano-4-iodo-phenyl)-N,N-dimethyl-formamidine (10.9g, 36.4mmol) was dissolved in dry THF (250ml) and diisopropyl borate (2eq, 16.8ml) added. The mixture was cooled to -78° and butyl lithium (1.6M in hexanes, 3eq, 69ml) added dropwise. The resulting dark yellow solution was stirred for a further 2h at -78° before being allowed to warm to room temperature. 2M HCl was added until the pH reached 6 then concentrated in vacuo to remove THF. The resulting solid material was isolated by filtration and washed with diethyl ether. This gave, after drying overnight in vacuo, the product as an off-white solid (7.6g, 95%)
¹H NMR (D₂O δ 8.40 (1H, s), 8.12 (1H, s), 8.04 (1H, d), 7.48 (1H, d), 3.40 (3H, s), 3.28 (3H, s)
LC-MS rt 0.4 m/z 217.96 ES+

### N'-{2-Cyano-4-[4-(4-morpholin-4-yl-phenylamino)-quinazolin-6-yl]-phenyl}-N,N-dimethyl-formamidine

A mixture of 3-Cyano-4-(dimethylamino-methyleneamino)-phenylboronic acid (184mg, 1.05eq) and (6-iodo-quinazolin-4-yl)-(4-morpholin-4-yl-phenyl)-amine (344mg, 0.8mmol) with tetrakistriphenylphosphine palladium (0) (5mol%, 45mg) in isopropanol (3.5ml) and saturated sodium bicarbonate (1.75ml) was heated to 100° for 2h then cooled and filtered. The resulting solid was washed with isopropanol (4x1ml) then TBME (2x1ml) and dried in vacuo to afford the title compound as a yellow solid ( 318mg, 83%)
¹H NMR (DMSO) δ 9.83 (1H, s), 8.79 (1H, s), 8.49 (1H, s), 8.21-8.0 (4H, m), 7.77 (1H, d) 7.64 (2H, d), 7.34 (1H, d) , 7.0 (2H, d), 3.76 (4H, m) 3.11 (7H,m), 3.03 (3H, s) ¹³C NMR (DMSO) δ 158.225, 155.349, 155.080, 154.796, 149.236, 148.185, 136.008, 132.521, 132.418, 131.419, 131.336, 131.086, 128.581, 124.510, 124.111, 119.884, 119.643, 119.057, 115.886, 115.425, 106.991, 66.483, 49.168, 40.229, 34.443
LC-MS rt 2.35 m/z 478

### N*4*-(4-Morpholin-4-yl-phenyl)-N*4'*-phenyl-[6,6']biquinazolinyl-4,4'-diamine trihydrochloride

N'-{2-Cyano-4-[4-(4-morpholin-4-yl-phenylamino)-quinazolin-6-yl]-phenyl}-N,N-dimethyl-formamidine (300mg) was treated with aniline (250uL) in acetic acid (2ml) at 125° for 2h. The cooled reaction mixture was neutralized with 2N NaOH and the resulting green precipitate isolated by filtration. This was then taken up in 2N HCl and concentrated to dryness. On standing with 1: 1 MeOH/EtOH an orange solid precipitated which was washed with acetone and ether to give the title compound as a red solid (170mg).
¹H NMR (DMSO) δ 12.56 (1H, s), 12.42 (1H, s), 10.38 (2H, m), 9.40 (2H, d), 9.214 ( 2H, m), 8.50 (2H, m), 8.36 (2H, d), 8.24 (2H, d), 7.85-7.73 (4H, m), 7.52 (2H, d), 4.25 (4H, m), 3.65 (4H, m)
LC-MS rt 2.52 m/z 526

### Example 3: N*4*,N*4'*-Bis-(4-[1,2,4]triazol-1-yl-phenyl)-[6,6']biquinazolinyl-4,4'-diamine

N'-[3,3'-Dicyano-4'-(dimethylamino-methyleneamino)-biphenyl-4-yl]-N,N-dimethylformamidine (100mg, 0.29mmol) and 4-(1H-1,2,4-triazol-1-yl)aniline (93mg, 0.58mmol) were combined in acetic acid (500uL) and heated to 125° for 90minutes. The solid was isolated by filtration and washed with water, then slurried with aqueous potassium carbonate. The solid was filtered and washed with water before drying in vacuo to give the title compound as a cream solid(90mg, 54%)
¹H NMR (DMSO) δ 11.95 (1H, s), 10.2 (1H, s), 9.28 (1H, s), 9.03 (1H, s) 8.69 (1H, S), 8.47 (1H, d) 8.25 (1H, s), 7.9-8.1 (4H, m)
LC-MS rt 2.43 m/z 573 ES- 575 ES+

### Activity Example

### Cells used:

HCV replicon cells Huh 9B (ReBlikon), containing the firefly luciferase - ubiquitin - neomycin phosphotransferase fusion protein and EMCV-IRES driven HCV polyprotein with cell culture adaptive mutations.

### Cell culture conditions:

Cells were cultured at 37°C in a 5% CO₂ environment and split twice a week on seeding at 2 x 10E6 cells/flask on day 1 and 1 x 10E6 3 days later. Some 0.25mg/ml G418 was added to the culture medium (125ul per 25ml) but not the assay medium.

The culture medium consisted of DMEM with 4500g/l glucose and glutamax (Gibco 61965-026) supplemented with 1 x non-essential amino acids, penicillin (100 IU/ml) / streptomycin (100 µg/ml), FCS (10%, 50ml) and 1 mg/ml G418 (Invitrogen cat no 10131-027) & 10 % foetal calf serum.

### Assay procedure:

A flask of cells was trypsinised and a cell count carried out. Cells were diluted to 100,000 cells/ml and 100 µl of this used to seed one opaque white 96-well plate (for the replicon assay) and one flat-bottomed clear plate (for the tox assay) for every seven compounds to be tested for IC50. Wells G12 and H12 were left empty in the clear plate as the blank. Plates were then incubated at 37°C in a 5% CO₂ environment for 24 h.

On the following day compound dilutions are made up in medium at twice their desired final concentration in a clear round bottomed plate. All dilutions have a final DMSO concentration of 1%.

Once the dilution plate had been made up, controls and compounds were transferred to the assay plate (containing the cells) at 100µl /well in duplicate plates. Exception: in the white (replicon) plate, no compound was added to wells A1 and A2 and 100 µl of 1% DMSO was added to these instead. In the clear (Tox) plate, wells E12 & F12 only contained the DMSO control. Plates were then incubated at 37°C with 5% CO₂ for 72h.

At the end of the incubation time, the cells in the white plate were harvested by washing with 200 µl/ well of warm (37°C) PBS and lysed with 20 µl cell culture lysis buffer (Promega). After 5 min incubation @ RT, luciferin solution was added to the luciferase assay buffer (LARB at 200 µl per 10 ml LARB. The M injector of the microplate luminometer (Lmax, Molecular Devices) was primed with 4 x 3001 injections. Plate were inserted into the luminometer and 100 µl luciferase assay reagent was added by the injector on the luminometer. The signal was measured using a 1 second delay followed by a 4 second measurement programme. The IC50, the concentration of the drug required for reducing the replicon level by 50% in relation to the untreated cell control value, can be calculated from the plot of the percentage reduction of the luciferase activity vs. drug concentration.

The clear plate was stained with 100 µl 0.5% methylene blue in 50% ethanol at RT for 1h, followed by solvation of the absorbed methylene blue in 100 µl per well of 1% lauroylsarcosine. Absorbance of the plate was measured on a microplate spectrophotometer (Molecular Devices) and the absorbance for each concentration of compound expressed as a proportion of the relative DMSO control. The TD50, the concentration of drug required to reduce the total cell area by 50% relative to the DMSO controls can be calculated by plotting the absorbance at 620nm vs drug concentration.

| | Replicon IC50 (<1 uM=***; 1 to 5uM=**; >10uM=*) | Replicon TD50 (>25uM=***; 10 to 25uM=**; <10uM=*) |
|---|---|---|
| Example no | uM | uM |
| 1 | *** | *** |
| 2 | *** | *** |
| 3 | *** | *** |

## Claims

1. A quinazoline derivative of formula (I), or a pharmaceutically acceptable salt thereof, 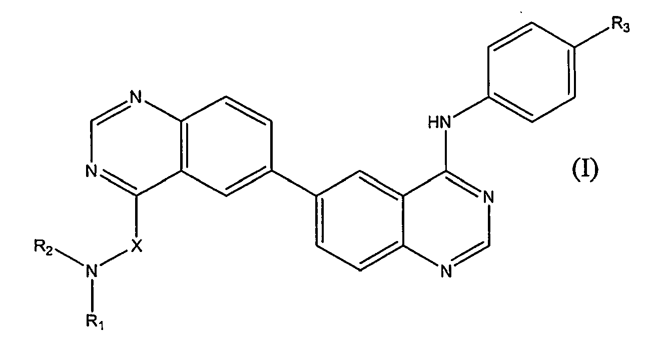 wherein:
- X represents a direct bond or a moiety -L-NR-, wherein R is hydrogen or C₁-C₄ alkyl, and L represents a C₁-C₄ alkylene, C₆-C₁₀ aryl or 5- to 10- membered heteroaryl moiety;
- either R₁ and R₂, together with the N atom to which they are attached, form a 5-to 10- membered heterocyclyl group or a 5- to 10- membered heteroaryl group, or R₁ represents hydrogen, C₆-C₁₀ aryl, C₁-C₄ alkyl or C₁-C₄ hydroxyalkyl and R₂ represents C₆-C₁₀ aryl, C₁-C₄ alkyl or C₁-C₄ hydroxyalkyl; and
- R₃ represents a C₆-C₁₀ aryl, C₃-C₆ carbocyclyl, 5- to 10- membered heteroaryl or 5- to 10- membered heterocyclyl moiety,
said aryl, carbocyclyl, heteroaryl and heterocyclyl groups being unsubstituted or substituted by 1, 2 or 3 substituents selected from halogen, C₁-C₄ alkyl, C₁-C₄ alkoxy, C₁-C₄ haloalkyl, C₁-C₄ haloalkoxy, hydroxy, thiol, -NH₂, C₁-C₄ hydroxyalkyl, C₁-C₄ thioalkyl and C₁-C₄ aminoalkyl substituents.

2. A compound according to claim 1, wherein the aryl, heteroaryl, carbocyclyl and heterocyclyl moieties in the R₁, R₂, R₃ and X moieties are unsubstituted or substituted by 1 or 2 substituents selected from halogen, C₁-C₄ alkyl and C₁-C₄ haloalkyl substituents.

3. A compound according to claim 1 or 2, wherein R is H.

4. A compound according to any one of the preceding claims, wherein L is a phenylene moiety.

5. A compound according to any one of the preceding claims, wherein X is -L-NR-, wherein L and R are as defined in any one of the preceding claims.

6. A compound according to any one of the preceding claims, wherein R₃ represents a 5- to 6- membered heteroaryl or heterocyclyl group.

7. A compound according to any one of the preceding claims, wherein when R₁ and R₂ do not together form a cyclic moiety, R₁ is hydrogen and R₂ is phenyl.

8. A compound according to any one of the preceding claims, wherein when R₁ and R₂ together form a cyclic moiety, R₁ and R₂, together with the N atom to which they are attached, form a 5- to 6- membered heterocyclyl or 5- to 6- membered heteroaryl group.

9. A compound according to claim 8 wherein R₁ and R₂, together with the N atom to which they are attached, form a saturated 5- to 6- membered heterocycle, which contains 1 or 2 heteroatoms selected from N, O and S.

10. A compound according to any one of the preceding claims, wherein the compound of formula (I) is *N*-(4-morpholinophenyl)-6-(4-(4-morpholinophenylamino)quinazolin-6-yl)quinazolin-4-amine, N*4*-(4-Morpholin-4-yl-phenyl)-N*4*-phenyl-[6,6']biquinazolinyl-4,4'-diamine or
N*4*,N*4'*-Bis-(4-[1,2,4]triazol-1-yl-phenyl)-[6,6']biquinazolinyl-4,4'-diamine

11. A quinazoline derivative of the formula (I), as defined in any one of the preceding claims, or a pharmaceutically acceptable salt thereof, for use in treating the human or animal body.

12. A pharmaceutical composition which comprises a quinazoline derivative of the formula (I), as defined in any one of claims 1 to 10, or a pharmaceutically acceptable salt thereof, and a pharmaceutically acceptable carrier or diluent.

13. Use of a quinazoline derivative of the formula (I), as defined in any one of claims 1 to 10, or a pharmaceutically acceptable salt thereof, in the manufacture of a medicament for use in treating or preventing a flaviviridae infection.

14. Use according to claim 13, wherein the flaviviridae infection is a pestivirus infection.

15. Use according to claim 14, wherein the pestivirus infection is an infection by a bovine viral diarrhea virus, classical swine fever virus or border disease virus.

16. Use according to claim 13, wherein the flaviviridae infection is a flavivirus infection.

17. Use according to claim 13, wherein the flavivirus infection is an infection by a yellow fever virus, dengue fever virus, Japanese encephalitis virus or tick borne encephalitis virus.

18. Use according to claim 13, wherein the flavivirdae infection is a hepacivirus infection.

19. Use according to claim 18, wherein the hepacivirus infection is an infection by a hepatitis C virus.

20. Use according to claim 19, wherein the medicament further comprises (a) interferon or a derivative thereof and/or (b) ribavirin or a derivative thereof.

21. Use according to claim 20 wherein the interferon derivative is PEG-interferon and/or the ribavirin derivative is viramidine.

22. A product containing:
(a) a quinazoline derivative of the formula (I), as defined in any one of claims 1 to 10, or a pharmaceutically acceptable salt thereof; and
(b) interferon, or an interferon derivative as defined in claim 20 or 21 and/or ribavirin or a ribavirin derivative as defined in claim 20 or 21;
for simultaneous, separate or sequential use in the treatment of the human or animal body.

23. A compound which is 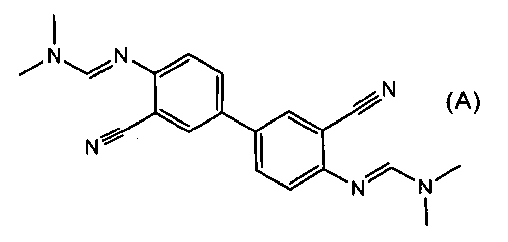 or

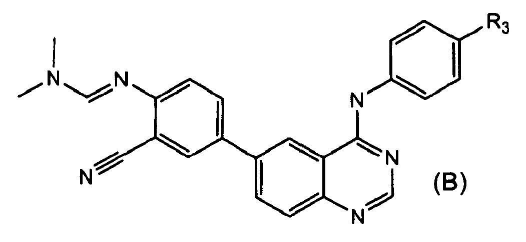

## Patentansprüche

1. Chinazolinderivate der Formel (I) und deren pharmazeutisch annehmbaren Salze, 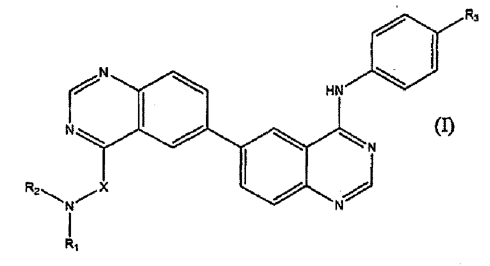 wobei:
- X für eine direkte Bindung oder eine -L-NR-Gruppe steht, wobei R für Wasserstoff oder C₁-C₄-Alkyl steht und L für eine C₁-C₄-Alkylengruppe, eine C₆-C₁₀-Arylgruppe oder eine 5- bis 10-gliedrige Heteroarylgruppe steht;
- entweder R₁ und R₂ zusammen mit dem Stickstoffatom, an das sie gebunden sind, eine 5- bis 10-gliedrige Heterocyclylgruppe oder eine 5- bis 10-gliedrige Heteroarylgruppe bilden oder R₁ für Wasserstoff, C₆-C₁₀-Aryl, C₁-C₄-Alkyl oder C₁-C₄-Hydroxyalkyl steht und R₂ für C₆-C₁₀-Aryl, C₁-C₄-Alkyl oder C₁-C₄-Hydroxyalkyl steht;
und
- R₃ für eine C₆-C₁₀-Arylgruppe, eine C₃-C₆-Carbocyclylgruppe, eine 5- bis 10-gliedrige Heteroarylgruppe oder eine 5- bis 10-gliedrige Heterocyclylgruppe steht,
wobei die Aryl-, Carbocyclyl-, Heteroaryl- und Heterocyclylgruppen unsubstituiert oder durch 1, 2 oder 3 Substituenten ausgewählt aus Halogen-, C₁-C₄-Alkyl-, C₁-C₄-Alkoxy-, C₁-C₄-Halogenalkyl-, C₁-C₄-Halogenalkoxy-, Hydroxy-, Thiol-, -NH₂-, C₁-C₄-Hydroxyalkyl-, C₁-C₄-Thioalkyl- und C₁-C₄-Aminoalkylsubstituenten substituiert sind.

2. Verbindung nach Anspruch 1, wobei die Aryl-, Heteroaryl-, Carbocyclyl- und Heterocyclylgruppen in den R₁-, R₂-, R₃- und X-Gruppen unsubstituiert oder durch 1 oder 2 Substituenten ausgewählt aus Halogen-, C₁-C₄-Alkyl- und C₁-C₄-Halogenalkylsubstituenten substituiert sind.

3. Verbindung nach Anspruch 1 oder 2, wobei R für H steht.

4. Verbindung nach einem der vorhergehenden Ansprüche, wobei L für eine Phenylengruppe steht.

5. Verbindung nach einem der vorhergehenden Ansprüche, wobei X für -L-NR- steht, wobei L und R wie in einem der vorhergehenden Ansprüche definiert sind.

6. Verbindung nach einem der vorhergehenden Ansprüche, wobei R₃ für eine 5- oder 6-gliedrige Heteroaryl- oder Heterocyclylgruppe steht.

7. Verbindung nach einem der vorhergehenden Ansprüche, wobei, wenn R₁ und R₂ zusammen keine zyklische Gruppe bilden, R₁ für Wasserstoff steht und R₂ für Phenyl steht.

8. Verbindung nach einem der vorhergehenden Ansprüche, wobei, wenn R₁ und R₂ zusammen eine zyklische Gruppe bilden, R₁ und R₂ zusammen mit dem Stickstoffatom, an das sie gebunden sind, eine 5- oder 6-gliedrige Heterocyclylgruppe oder eine 5- oder 6-gliedrige Heteroarylgruppe bilden.

9. Verbindung nach Anspruch 8, wobei R₁ und R₂ zusammen mit dem Stickstoffatom, an das sie gebunden sind, einen gesättigten 5- oder 6-gliedrigen Heterozyklus bilden, der 1 oder 2 Heteroatome ausgewählt aus N, 0 und S enthält.

10. Verbindungen nach einem der vorhergehenden Ansprüche, wobei es sich bei der Verbindung der Formel (I) um *N*-(4-Morpholinophenyl)-6-(4-(4-morpholinophenylamino)chinazolin-6-yl)chinazolin-4-amin, N*4*-(4-Morpholin-4-yl-phenyl)-N*4*-phenyl-[6,6']bichinazolinyl-4,4'-diamin oder N*4*,N*4'*-Bis-(4-[1,2,4]triazol-1-yl-phenyl)-[6,6']bichinazolinyl-4,4'-diamin handelt.

11. Chinazolinderivat der Formel (I) nach einem der vorhergehenden Ansprüche oder ein pharmazeutisch annehmbares Salz davon zur Verwendung bei der Behandlung des menschlichen oder tierischen Körpers.

12. Pharmazeutische Zusammensetzung, enthaltend ein Chinazolinderivat der Formel (I) nach einem der Ansprüche 1 bis 10 oder ein pharmazeutisch annehmbares Salz davon und einen pharmazeutisch annehmbaren Träger oder ein pharmazeutisch annehmbares Verdünnungsmittel.

13. Verwendung eines Chinazolinderivats der Formel (I) nach einem der Ansprüche 1 bis 10 oder eines pharmazeutisch annehmbaren Salzes davon bei der Herstellung eines Medikaments zur Behandlung oder Prävention einer Flaviviridae-Infektion.

14. Verwendung nach Anspruch 13, wobei es sich bei der Flaviviridae-Infektion um eine Pestivirus-Infektion handelt.

15. Verwendung nach Anspruch 14, wobei es sich bei der Pestivirus-Infektion um eine Infektion durch eine Bovine-Virusdiarrhoe-Virus, Virus der klassischen Schweinepest oder Border-Disease-Virus handelt.

16. Verwendung nach Anspruch 13, wobei es sich bei der Flaviviridae-Infektion um eine Flavivirus-Infektion handelt.

17. Verwendung nach Anspruch 13, wobei es sich bei der Flavivirus-Infektion um eine Infektion durch ein Gelbfiebervirus, Dengue-Fieber-Virus, Japanische-Enzephalitis-Virus oder ein von Zecken übertragenes Enzephalitis-Virus handelt.

18. Verwendung nach Anspruch 13, wobei es sich bei der Flaviviridae-Infektion um eine Hepacivirus-Infektion handelt.

19. Verwendung nach Anspruch 18, wobei es sich bei der Hepacivirus-Infektion um eine Infektion durch ein Hepatitis-C-Virus handelt.

20. Verwendung nach Anspruch 19, wobei das Medikament weiterhin (a) Interferon oder ein Derivat davon und/oder (b) Ribavirin oder ein Derivat davon enthält.

21. Verwendung nach Anspruch 20, wobei es sich bei dem Interferonderivat um PEG-Interferon und/oder bei dem Ribavirinderivat um Viramidin handelt.

22. Produkt enthaltend:
(a) ein Chinazolinderivat der Formel (I) nach einem der Ansprüche 1 bis 10 oder ein pharmazeutisch annehmbares Salz davon;
(b) Interferon oder ein Interferonderivat nach Anspruch 20 oder 21 und/oder Ribavirin oder ein Ribavirinderivat nach Anspruch 20 oder 21;
zur gleichzeitigen, getrennten oder aufeinanderfolgenden Verwendung bei der Behandlung des menschlichen oder tierischen Körpers.

23. Verbindung, bei der es sich um 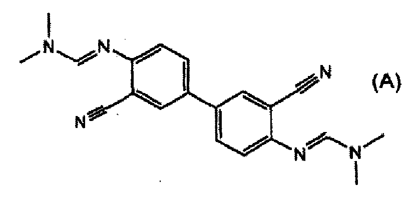 oder 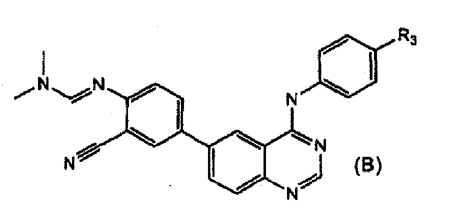 handelt.

## Revendications

1. Dérivé de la quinazoline de formule (I), ou un sel pharmaceutiquement acceptable de celui-ci, 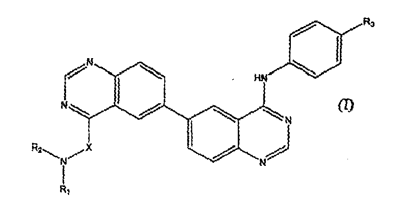 dans laquelle :
- X représente une liaison directe ou un motif - L-NR-, où R est hydrogène ou C₁-C₄ alkyle, et L représente un motif C₁-C₄ alkylène, C₆-C₁₀ aryle ou hétéroaryle à 5 à 10 chaînons ;
- soit R₁ et R₂, conjointement avec l'atome de N auquel ils sont liés, forment un groupement hétérocyclyle à 5 à 10 chaînons ou un groupement hétéroaryle à 5 à 10 chaînons, soit R₁ représente hydrogène, C₆-C₁₀ aryle, C₁-C₄ alkyle ou C₁-C₄ hydroxyalkyle et R₂ représente C₆-C₁₀ aryle, C₁-C₄ alkyle ou C₁-C₄ hydroxyalkyle ; et
- R₃ représente un motif C₆-C₁₀ aryle, C₃-C₆ carbocyclyle, hétéroaryle à 5 à 10 chaînons ou hétérocyclyle à 5 à 10 chaînons,
lesdits groupements aryle, carbocyclyle, hétéroaryle et hétérocyclyle étant non substitués ou substitués par 1, 2 ou 3 substituants choisis parmi les substituants halogène, C₁-C₄ alkyle, C₁-C₄ alcoxy, C₁-C₄ halogéno-alkyle, C₁-C₄ halogéno-alcoxy, hydroxy, thiol, -NH₂, C₁-C₄ hydroxyalkyle, C₁-C₄ thioalkyle et C₁-C₄ amino-alkyle.

2. Composé selon la revendication 1, **caractérisé en ce que** les motifs aryle, hétéroaryle, carbocyclyle et hétérocyclyle dans les motifs R₁, R₂, R₃ et X sont non substitués ou substitués par 1 ou 2 substituants choisis parmi les substituants halogène, C₁-C₄ alkyle et C₁-C₄ halogéno-alkyle.

3. Composé selon la revendication 1 ou 2, **caractérisé en ce que** R est H.

4. Composé selon l'une quelconque des revendications précédentes, **caractérisé en ce que** L est un motif phénylène.

5. Composé selon l'une quelconque des revendications précédentes, **caractérisé en ce que** X est -L-NR-, où L et R sont tels que définis dans l'une quelconque des revendications précédentes.

6. Composé selon l'une quelconque des revendications précédentes, **caractérisé en ce que** R₃ représente un groupement hétéroaryle ou hétérocyclyle à 5 à 6 chaînons.

7. Composé selon l'une quelconque des revendications précédentes, **caractérisé en ce que** lorsque R₁ et R₂ ensemble ne forment pas un motif cyclique, R₁ est hydrogène et R₂ est phényle.

8. Composé selon l'une quelconque des revendications précédentes, **caractérisé en ce que** lorsque R₁ et R₂ ensemble forment un motif cyclique, R₁ et R₂, conjointement avec l'atome de N auquel ils sont liés forment un groupement hétérocyclyle à 5 à 6 chaînons ou hétéroaryle à 5 à 6 chaînons.

9. Composé selon la revendication 8, **caractérisé en ce que** R₁ et R₂, conjointement avec l'atome de N auquel ils sont liés, forment un hétérocycle saturé à 5 à 6 chaînons, qui contient 1 ou 2 hétéroatomes choisis parmi N, 0 et S.

10. Composé selon l'une quelconque des revendications précédentes, **caractérisé en ce que** le composé de formule (I) est la N-(4-morpholinophényl)-6-(4-(4-morpholinophénylamino)quinazolin-6-yl)quinazolin-4-amine, la N*4*-(4-morpholin-4-yl-phényl)-N*4*-phényl-[6,6']biquinazolinyl-4,4'-diamine ou la N*4*,N*4'*-bis-(4-[1,2,4]triazol-1-yl-phényl)-[6,6']biquinazolinyl-4,4'diamine.

11. Dérivé de la quinazoline de formule (I), tel que défini dans l'une quelconque des revendications précédentes, ou un sel pharmaceutiquement acceptable de celui-ci, destiné à être utilisé dans le traitement de l'organisme humain ou animal.

12. Composition pharmaceutique, **caractérisée en ce qu'**elle comprend un dérivé de la quinazoline de formule (I), tel que défini dans l'une quelconque des revendications 1 à 10, ou un sel pharmaceutiquement acceptable de celui-ci, et un support ou un diluant pharmaceutiquement acceptable.

13. Utilisation d'un dérivé de la quinazoline de formule (I), tel que défini dans l'une quelconque des revendications 1 à 10, ou d'un sel pharmaceutiquement acceptable de celui-ci, dans la fabrication d'un médicament destiné à être utilisé dans le traitement ou la prévention d'une infection à flaviviridae.

14. Utilisation selon la revendication 13, **caractérisée en ce que** l'infection à flaviviridae est une infection à pestivirus.

15. Utilisation selon la revendication 14, **caractérisée en ce que** l'infection à pestivirus est une infection par un virus de la diarrhée virale bovine, un virus de la peste porcine classique ou un virus de la maladie de border.

16. Utilisation selon la revendication 13, **caractérisée en ce que** l'infection à flaviviridae est une infection à flavivirus.

17. Utilisation selon la revendication 13, **caractérisée en ce que** l'infection à flavivirus est une infection par un virus de la fièvre jaune, un virus de la dengue, un virus de l'encéphalite japonaise ou un virus de l'encéphalite à tiques.

18. Utilisation selon la revendication 13, **caractérisée en ce que** l'infection à flaviviridae est une infection à hépacivirus.

19. Utilisation selon la revendication 18, **caractérisée en ce que** l'infection à hépacivirus est une infection par un virus de l'hépatite C.

20. Utilisation selon la revendication 19, **caractérisée en ce que** le médicament comprend en outre (a) l'interféron ou un dérivé de celui-ci et/ou (b) la ribavirine ou un dérivé de celle-ci.

21. Utilisation selon la revendication 20, **caractérisée en ce que** le dérivé d'interféron est le PEG-interféron et/ou le dérivé de la ribavirine est la viramidine.

22. Produit contenant:
(a) un dérivé de la quinazoline de formule (I), tel que défini dans l'une quelconque des revendications 1 à 10, ou un sel pharmaceutiquement acceptable de celui-ci ; et
(b) l'interféron, ou un dérivé d'interféron tel que défini dans la revendication 20 ou 21 et/ou la ribavirine ou un dérivé de la ribavirine tel que défini dans la revendication 20 ou 21 ;
destiné à une utilisation simultanée, séparée ou séquentielle dans le traitement de l'organisme humain ou animal.

23. Composé, **caractérisé en ce qu'**il s'agit de 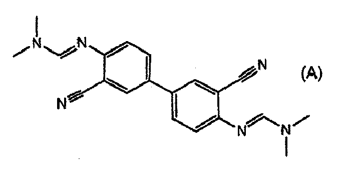 ou
